# EUROPEAN PATENT APPLICATION

(11) **EP 2 127 598 A1**
(43) Date of publication of application: **02.12.2009**
(21) Application number: 09006958.4
(22) Date of filing: 25.05.2009
(51) Int. Cl.: A61B 5/07, A61B 1/05, A61B 5/00

(54) **Capsule medical apparatus and method of charging capsule medical apparatus**

(30) Priority: 26.05.2008 JP 2008136731
(71) Applicant: Olympus Medical Systems Corporation, Tokyo 151-0072 (JP)
(72) Inventor: Kimoto, Seiichiro, Tokyo 151-0072 (JP)
(74) Representative: von Hellfeld, Axel

(57) **Abstract**

A capsule medical apparatus (1) includes a function executing unit (15), a secondary battery (10), a power input unit (11), and a connecting circuit (12). The function executing unit (15) performs a predetermined function. The secondary battery (10) supplies electric power to the function executing unit (15). The power input unit (11) receives electric power to charge the secondary battery (10). The connecting circuit (12) releasably connects the secondary battery (10) and the power input unit (11), and releases a connection between the secondary battery (10) and the power input unit (11) to inhibit charging the secondary battery (10).

## Description

### TECHNICAL FIELD

The present invention relates to a capsule medical apparatus that is introduced into the organs inside a subject, such as a patient, to acquire in-vivo information of the subject. The present invention also relates to a method of charging a capsule medical apparatus.

### BACKGROUND ART

Swallowable capsule medical apparatuses that have imaging and wireless-communication functions are used in the medical field. Usually for internal observation of a subject, a capsule medical apparatus is swallowed by a subject and, as it moves thorough the organs of the subject, it sequentially takes images of the interior of the organs of the subject and wirelessly transmits signals of the images to the outside of the subject. Images of the interior of the organs can be referred to as in-vivo images. The capsule medical apparatus repeatedly taking in-vivo images of the subject and wirelessly transmits signals of the images until it is naturally excreted by the subject.

The signals of the images that are wirelessly transmitted by the capsule medical apparatus inside the subject are received by a receiving device outside the subject. The receiving device includes receiving antennas that are arranged on the body surface of the subject. The receiving device receives the signals of the images from the capsule medical apparatus through the receiving antennas. A predetermined recording medium is previously attached to the receiving device. The receiving device then sequentially records to the recording medium the in-vivo images of the subject received from the capsule medical apparatus.

After the capsule medical apparatus is naturally excreted by the subject, the recording medium is detached from the receiving device and attached to a predetermined image display device. The image display apparatus reads an image-data group, i.e., a group of in-vivo images taken by the capsule medical apparatus, from the recording medium and displays the image-data group on a display. A user, such as a doctor or a nurse, can diagnose the subject by observing the group of in-vivo images displayed on the image display device.

Most capsule medical apparatuses incorporate button-shaped primary batteries as power supply units; however, some incorporate rechargeable secondary batteries (accumulator batteries) as power supply units (see Japanese Patent Application Laid-open No. 2002-306491). Capsule-type medical apparatuses use electric power from incorporated primary or secondary batteries to take in-vivo images and transmit signals of the images.

Before they are used, capsule medical apparatuses that are introduced into subjects, such as patients, usually undergo a sterilization process (e.g., after manufacture or when they are stored or shipped). Subjects introduce sterilized capsule medical apparatuses into themselves. It is desirable that used capsule medical apparatuses having been excreted by subjects are not introduced into subjects again but are collected and discarded. In other words, it is desired that a capsule medical apparatus is not unintentionally used again after being used for in-vivo examination of a subject, i.e., the number of times a capsule medical apparatus is used is limited to once.

### DISCLOSURE OF INVENTION

A capsule medical apparatus according to an aspect of the present invention includes a function executing unit that executes a predetermined function; a secondary battery that supplies electric power to the function executing unit; a power input unit in which electric power to charge the secondary battery is input; and a connecting circuit that releasably connects the secondary battery and the power input unit to each other. The connecting circuit releases a connection between the secondary battery and the power input unit to inhibit charging the secondary battery.

A method according to another aspect of the present invention is for charging a capsule medical apparatus that includes a function executing unit that executes a predetermined function; a secondary battery that supplies electric power to the function executing unit; a power input unit in which electric power to charge the secondary battery is input by receiving an external energy; and a fuse that connects the secondary battery and the power input unit. The method includes charging the secondary battery by applying an external energy to the capsule medical apparatus; detecting that charging the secondary battery is complete; and disconnecting the fuse to inhibit charging the secondary battery, when charging the secondary battery is complete.

A method according to still another aspect of the present invention is for charging a capsule medical apparatus that includes a function executing unit that performs a predetermined function; a secondary battery that supplies electric power to the function executing unit; a power input unit in which electric power to charge the secondary battery is input by receiving an external energy; and a semiconductor switching device that connects the secondary battery and the power input unit. The method includes charging the secondary battery by applying a predetermined external energy to the capsule medical apparatus; detecting that charging the secondary battery is complete; and causing the semiconductor switching device to open to inhibit charging the secondary battery, when charging the secondary battery is complete.

The above and other features, advantages and technical and industrial significance of this invention will be better understood by reading the following detailed description of presently preferred embodiments of the invention, when considered in connection with the accompanying drawings.

### BRIEF DESCRIPTION OF DRAWINGS

FIG. 1 is a schematic cross-sectional view of a configuration example of a capsule medical apparatus according to a first embodiment of the present invention.
FIG. 2 is a schematic block diagram of a functional configuration example of the capsule medical apparatus according to the first embodiment of the present invention.
FIG. 3 is a schematic diagram of an example of the state where the capsule medical apparatus according to the first embodiment of the present invention is charged.
FIG. 4 is a schematic block diagram of a functional configuration example of a capsule medical apparatus according to Modification 1 of the first embodiment.
FIG. 5 is a schematic block diagram of a functional configuration example of a capsule medical apparatus according to Modification 2 of the first embodiment.
FIG. 6 is a schematic block diagram of a functional configuration example of a capsule medical apparatus according to a second embodiment of the present invention.
FIG. 7 is a schematic block diagram of a functional configuration example of a capsule medical apparatus according to Modification 1 of the second embodiment.
FIG. 8 is a schematic block diagram of a functional configuration example of a capsule medical apparatus according to Modification 2 of the second embodiment.
FIG. 9 is a schematic block diagram of a functional configuration example of a capsule medical apparatus according to a third embodiment of the present invention.
FIG. 10 is a flowchart of a procedure performed by a power-supply controller of the capsule medical apparatus according to the third embodiment of the present invention.
FIG. 11 is a schematic diagram representing how the capsule medical apparatus according to the third embodiment of the present invention is introduced into a subject from the mouth and excreted by the subject.
FIG. 12 is a block diagram of an example of another arrangement of a fuse of a power supply unit of the capsule medical apparatus according to the first embodiment of the preset invention.
FIG. 13 is a block diagram of an example of another arrangement of a fuse of a power supply unit of the capsule medical apparatus according to Modification 1 of the first embodiment.

### BEST MODE(S) FOR CARRYING OUT THE INVENTION

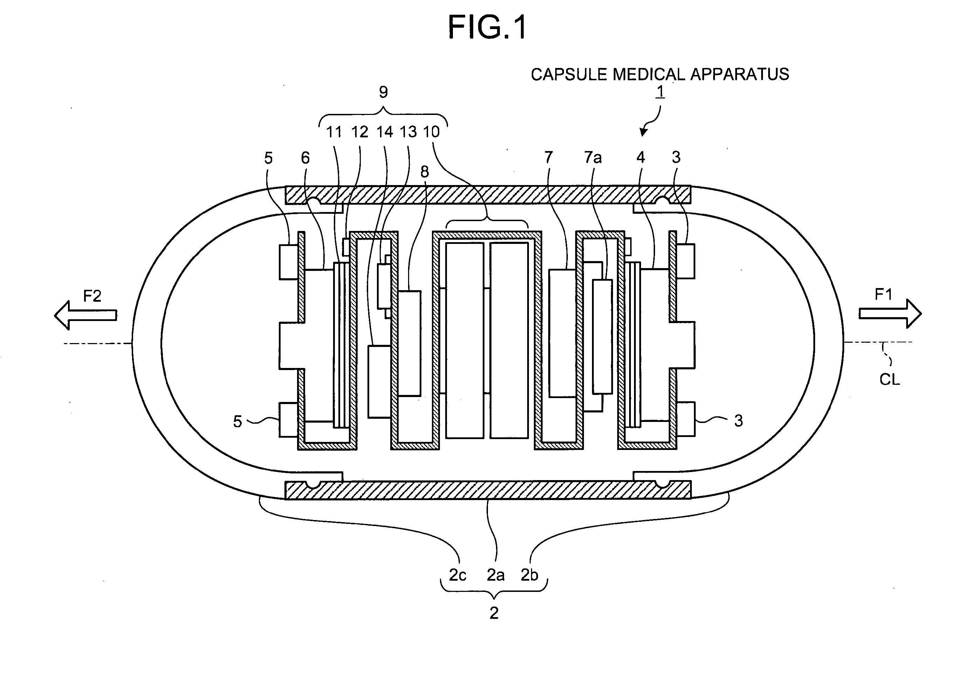

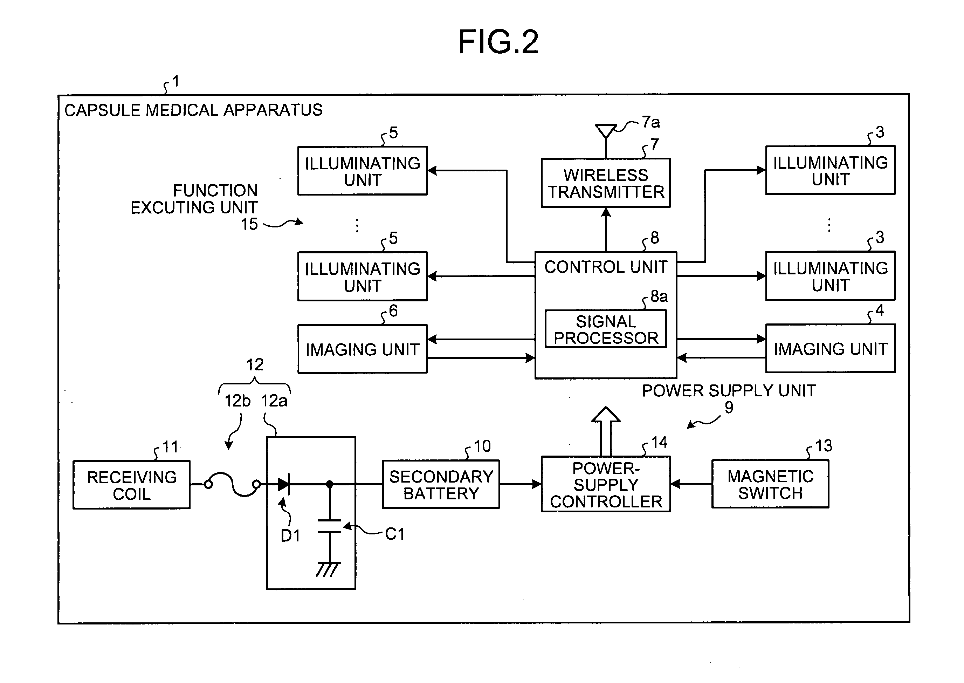

FIG. 1 is a cross-sectional schematic diagram of a configuration example of a capsule medical apparatus according to a first embodiment of the present invention. FIG. 2 is a schematic block diagram of a functional configuration example of the capsule medical apparatus according to the first embodiment of the present invention. As shown in FIGS. 1 and 2, a capsule medical apparatus 1 according to the first embodiment of the present invention includes a capsule casing 2 in a size that can be introduced into a subject; illuminating units 3 and 5 that illuminate the interior of the subject in different directions; an imaging unit 4 that takes images of objects illuminated by the illuminating unit 3; and an imaging unit 6 that takes in-vivo images of objects illuminated by the illuminating unit 5. The capsule medical apparatus 1 further includes a wireless transmitter 7 that wirelessly transmits in-vivo images taken by the imaging units 4 and 6; a control unit 8 that controls the units constituting the capsule medical apparatus 1; and a power supply unit 9 that is rechargeable and supplies electric power to the units constituting the capsule medical apparatus 1.

The capsule casing 2 is in a size that can be easily introduced into the organs of a subject from, for example, the mouth. As shown in FIG. 1, the capsule casing 2 has dome-shaped end portions and a cylindrical body portion. Both open ends of a cylindrical casing 2a of the capsule casing 2, which serves as the body portion, are closed respectively with dome-shaped casings 2b and 2c. The cylindrical casing 2a is impenetrable by a visible light, and the dome-shaped casings 2b and 2c are penetrable by a visible light. The capsule casing 2 formed of the cylindrical casing 2a and the dome-shaped casings 2b and 2c houses watertight the units constituting the capsule medical apparatus 1, specifically, the illuminating unit 3 and 5, the imaging units 4 and 6, the wireless transmitter 7, the control unit 8, and the power supply unit 9.

The illuminating units 3 are, for example, light emitting devices such as LEDs. The illuminating unit 3 emits lights in a predetermined wavelength band to illuminate the interior of the subject, which is the object of the imaging unit 4, through the dome-shaped casing 2b.

The imaging unit 4 functions as an in-vivo information acquiring unit that acquires in-vivo images that are an example of in-vivo information of the subject. Specifically, the imaging unit 4 includes a solid-state imaging device, such as a CCD or a CMOS image sensor, and an optical system. The imaging unit 4 focuses the light that is reflected from an object illuminated by the illuminating unit 3, so that an optical image of the object is formed. The imaging unit 4 receives the optical image of the subject, using the solid-state imaging device, so that an image of the subject, i.e., an image of the interior of an organ on the side of the direction F1 is taken. The direction F1 is the direction in which the imaging unit 4 takes images, and is the direction on the side of the dome-shaped casing 2b defined by the center axis CL in the longitudinal direction of the capsule casing 2.

The illuminating units 5 are, for example, light emitting devices such as LEDs. The illuminating units 5 emit light in a predetermined wavelength band to illuminate the interior of the subject (specifically, interior of organs), which is the object of the imaging unit 6, through the dome-shaped casing 2c.

The imaging unit 6 functions as an in-vivo information acquiring unit that acquires in-vivo images that are an example of in-vivo information of the subject. Specifically, the imaging unit 6 includes a solid-state imaging device, such as a CCD or a CMOS image sensor, and an optical system. The imaging unit 6 focuses the light that is reflected from an object illuminated by the illuminating units 5, so that an optical image of the object is formed. The imaging unit 6 receives the optical image of the subject, using the solid-state imaging device, so that an image of the subject, i.e., the image of the interior of an organ on the side of the direction F2 is taken. The direction F2 is the direction in which the imaging unit 6 takes images, and is the direction on the side of the dome-shaped casing 2c defined by the center axis CL in the longitudinal direction of the capsule casing 2.

The wireless transmitter 7 wirelessly transmits the in-vivo information of the subject to the outside. Specifically, the wireless transmitter 7 includes a coil-shaped or loop-shaped transmitting antenna 7a. The wireless transmitter 7 sequentially receives signals of the in-vivo images taken by the imaging units 4 and 6, performs a predetermined modulating process on the signals of the in-vivo images to generate wireless signals containing the in-vivo images taken by the imaging unit 4 or 6. The wireless transmitter 7 sequentially transmits the wireless signals containing the in-vivo images to the outside through the transmitting antenna 7a. The wireless signals containing the in-vivo images of the subject transmitted by the wireless transmitter 7 are received by the external receiving device that is, for example, carried by the subject.

The control unit 8 controls operations of the units constituting the capsule medical apparatus 1, i.e., the illuminating units 3 and 5, the imaging units 4 and 6, and the wireless transmitter 7, and controls input and output of signals between the units. Specifically, the control unit 8 controls the operation timing of the illuminating unit 3 and the imaging unit 4 such that in-vivo images of the side of the direction F1 illuminated by the illuminating unit 3 are taken, and controls the operation timing of the illuminating unit 5 and the imaging unit 6 such that in-vivo images on the side of the direction F2 illuminated by the illuminating unit 5 are taken. The control unit 8 further includes a signal processor 8a. The signal processor 8a has various types of parameters concerning the image processing on, such as white balance, and generates signals of in-vivo images taken by the imaging units 4 and 6. The control unit 8 sequentially sends the signals of the in-vivo images to the wireless transmitter 7, and controls the wireless transmitter 7 such that it sequentially transmits wireless signals containing the in-vivo images to the outside.

The illuminating units 3 and 5, the imaging units 4 and 6, the wireless transmitter 7, and the control unit 8 constitute a function executing unit 15 that executes predetermined functions. The functions executed by the function executing unit 15 include the function of illuminating an object, which is performed by the illuminating units 3 and 5; the function of taking in-vivo images, which is performed by the imaging units 4 and 6; and the function of wirelessly transmitting in-vivo images, which is performed by the wireless transmitter 7. The units constituting the function executing unit 15 and the power supply unit 9 are electrically connected to one another in the capsule casing 2 through, for example, a rigid circuit board or a flexible circuit board.

The power supply unit 9 is rechargeable with external energy, such as a magnetic field applied from the outside, and supplies electric power stored therein by the charging to the function executing unit 15. Specifically, the power supply unit 9 includes a rechargeable secondary battery 10; a receiving coil 11 to which electric power to charge the secondary battery 10 is input; a connecting circuit 12 that releasably connects the secondary battery 10 and the receiving coil 11, a magnetic switch 13 that switches ON/OFF the power supply unit 9, and a power-supply controller 14 that controls supply of electric power to the function executing unit 15.

The secondary battery 10 supplies electric power to the function executing unit 15. Specifically, the secondary battery 10 is electrically connected to the receiving coil 11 through the connecting circuit 12, and receives the electric power generated by the receiving coil 11 through the connecting circuit 12. Thus, electric power necessary for operations of the function executing unit 15 is stored in the secondary battery 10. The secondary battery 10 having been charged supplies electric power to the units constituting the function executing unit 15 under the control of the power-supply controller 14.

The number of secondary batteries is not limited to two. It may be any number including one as long electric power necessary for operations of the function executing unit 15 can be stored. The secondary battery 10 may be button-shaped, platy, or sheet-shaped.

The receiving coil 11 functions as a power input unit that inputs electric power to charge the secondary battery 10, i.e., charging power. Specifically, the receiving coil 11 receives an external magnetic field applied by an external charging device (not shown), and converts the external magnetic field to electric power. The receiving coil 11 supplies the electric power, which is based on the magnetic field, to the secondary battery 10 through the connecting circuit 12.

The connecting circuit 12 releasably connects the secondary battery 10 and the receiving coil 11. Specifically, the connecting circuit 12 includes a rectifier circuit 12a that rectifies a current of the electric power generated in the receiving coil 11; and a fuse 12b that connects the secondary battery 10 and the receiving coil 11.

The rectifier circuit 12a includes a diode D1 and a capacitor C1, and forms a part of a conduction path between the secondary battery 10 and the receiving coil 11. The diode D1 is arranged such that the current direction from the receiving coil 11 toward the secondary battery 10 is the forward direction of the diode. One terminal of the capacitor C1 is connected to an output terminal of the diode D1, and the other terminal of the capacitor C1 is connected to a ground potential. The rectifier circuit 12a performs rectification such that the current direction of the conduction path between the secondary battery 10 and the receiving coil 11 is from the receiving coil 11 toward the secondary battery 10.

The fuse 12b releasably connects the secondary battery 10 and the receiving coil 11. Specifically, one terminal of the fuse 12b is connected to the receiving coil 11, and the other terminal of the fuse 12b is connected to an input terminal of the diode D1 of the rectifier circuit 12a, which forms a part of the conduction path between the secondary battery 10 and the receiving coil 11, i.e., other parts of the conduction path excluding the part formed by the rectifier circuit 12a. When the current of the electric power from the receiving coil 11 is smaller than a predetermined value, the fuse 12b maintains the state where the secondary battery 10 and the receiving coil 11 are connected. In contrast, when the current of the electric power from the receiving coil 11 is equal to or larger than the predetermined value, the fuse 12b is disconnected, so that the connection between the secondary battery 10 and the receiving coil 11 is released. In this manner the fuse 12b releases the connection between the secondary battery 10 and the receiving coil 11, thereby inhibiting charging the secondary battery 10.

The magnetic switch 13 switches ON/OFF the power supply unit 9. Specifically, when a magnetic signal in a predetermined pattern is applied from the outside, the magnetic switch 13 receives the magnetic signal of the pattern, and then sends, to the power-supply controller 14, an instruction signal for starting supplying electric power to the function executing unit 15 based on the received magnetic signal. In other words, the magnetic switch 13 functions as an instructing unit that issues an instruction for starting supplying the electric power from the secondary battery 10 to the function executing unit 15.
When a magnetic signal of a pattern that is different from that of the magnetic signal serving as an instruction for starting supplying electric power is applied, the magnetic switch 13 may receive the magnetic signal of the different pattern and transmit, to the power-supply controller 14, an instruction signal for stopping supplying electric power to the function executing unit 15 based on the magnetic signal.

The power-supply controller 14 controls ON/OFF of the power supply unit 9. Specifically, in the initial state before the instruction signal for starting supplying electric power is input from the magnetic switch 13, the power-supply controller 14 maintains the state where supplying the electric power from the secondary battery 10 to the function executing unit 15 is stopped, i.e., the OFF state. In contrast, when the power-supply controller 14 receives the instruction signal for starting supplying electric power from the magnetic switch 13, it starts to supply the electric power from the secondary battery 10 to the function executing unit 15 based on the received instruction signal. Thereafter, the power-supply controller 14 maintains the where the supply of the electric power from the secondary battery 10 to the function executing unit 15 is started, i.e., the ON state. During the ON state, the electric power from the secondary battery 10 is supplied to the units constituting the function executing unit 15. The power-supply controller 14 may stop supplying the electric power from the secondary battery 10 to the function executing unit 15 based on the received instruction signal based on an instruction signal received for stopping to supply electric power from the magnetic switch 13.

A method of charging the capsule medical apparatus 1 and inhibition of the charging according to the first embodiment of the present invention are explained below, taking the case where the capsule medical apparatus 1 incorporates the secondary battery 10 that is not recharged.

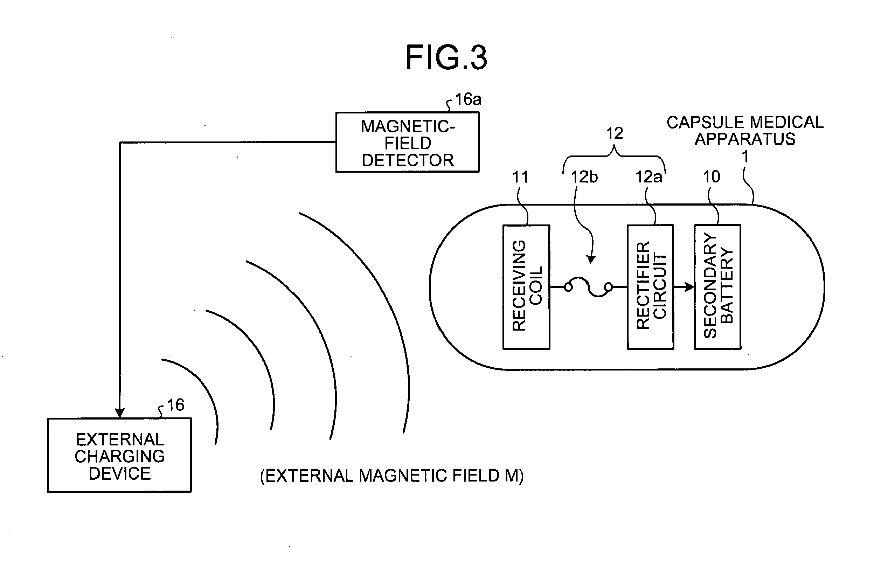

FIG. 3 is a schematic diagram of an example of the state where the capsule medical apparatus 1 according to the first embodiment of the present invention is charged. To simplify the explanation, only the secondary battery 10, the receiving coil 11, the rectifier circuit 12a, and the fuse 12b of the units incorporated in the capsule medical apparatus 1 are shown in FIG. 3.

As shown in FIG. 3, the capsule medical apparatus 1 incorporates the secondary battery 10 that is uncharged, the receiving coil 11 to which electric power to charge the secondary battery 10 is input, and the connecting circuit 12 that releasably connects the secondary battery 10 and the receiving coil 11. The secondary battery 10 is charged by an external charging device 16 just before the user, such as a doctor or nurse, uses the capsule medical apparatus 1, for example, before the capsule medical apparatus 1 is introduced into the subject.

The external charging device 16 charges the secondary battery 10 inside the capsule medical apparatus 1 by applying an external magnetic field M to the capsule medical apparatus 1. The external charging device 16 includes a magnetic-field detector 16a that detects the external magnetic field M applied to the capsule medical apparatus 1, and the external charging device 16 determines whether charging the secondary battery 10 is complete based on a result the detection by the magnetic-field detector 16a. The magnetic field strength of the external magnetic field M can be adjusted to a desired strength by the external charging device 16.

After the external magnetic field M is applied from the external charging device 16 to the capsule medical apparatus 1, the receiving coil 11 converts the applied external magnetic field M to electric power to charge the secondary battery 10. The current generated in the receiving coil 11 is not enough to disconnect the fuse 12b. The electric power generated in the receiving coil 11 is supplied to the secondary battery 10 through the fuse 12b and the rectifier circuit 12a. Accordingly, the secondary battery 10 stores therein electric power necessary for operations of the function executing unit 15, i.e., enters the charged state. In this manner, the secondary battery 10 of the capsule medical apparatus 1 is charged.

In addition to applying the external magnetic field M to the capsule medical apparatus 1, the external charging device 16 monitors the magnetic field strength of the external magnetic field M near the capsule medical apparatus 1, using the magnetic-field detector 16a. When charging the secondary battery 10 of the capsule medical apparatus 1 is not complete, there is a difference equal to or larger than a predetermined value between the magnetic field strength of the external magnetic field M that is applied by the external charging device 16 and the magnetic field strength detected by the magnetic-field detector 16a. In this case, the external charging device 16 determines that charging the secondary battery 10 is not complete based on the difference in magnetic field intensity, and maintains the state where the external magnetic field M is applied to the capsule medical apparatus 1. In contrast, when charging the secondary battery 10 is complete, the difference between the magnetic field strength of the external magnetic field M and the magnetic field strength detected by the magnetic-field detector 16a is smaller than the predetermined value. In this case, the external charging device 16 determines that charging the secondary battery 10 is complete based on the difference in magnetic field strength, and sets the magnetic field intensity of the external magnetic field M to the capsule medical apparatus to a value equal to or larger than a predetermined value.

When the external charging device 16 applies the external magnetic field M having the magnetic field intensity equal to or larger than the predetermined value to the capsule medical apparatus 1, the receiving coil 11 receives the external magnetic field M and generates electric power having a current equal to or larger than the predetermined value. The current generated in the receiving coil 11 is large enough to disconnect the fuse 12b. The electric power generated in the receiving coil 11 is input to the fuse 12b, and the fuse 12b is disconnected by the overcurrent of the electric power. The fuse 12b releases the connection between the secondary battery 10 and the receiving coil 11 in this manner, thereby inhibiting supplying electric power from the receiving coil 11 to the secondary battery 10, i.e., inhibiting charging the secondary battery 10.

After charging the secondary battery 10 of the capsule medical apparatus 1 is inhibited due to the disconnection of the fuse 12b, the secondary battery 10 cannot be recharged. In other words, the number of times the secondary battery 10 of the capsule medical apparatus 1 is charged is limited to once. After the charging is complete, the capsule medical apparatus 1 is switched ON by the magnetic switch 13, i.e., starts to supply the electric power from the secondary battery 10 to the function executing unit 15. Thereafter, the capsule medical apparatus 1 is introduced into the subject. In the subject, the capsule medical apparatus 1 executes the functions of the function executing unit 15 by using the electric power from the secondary battery 10. When the electric power from the secondary battery 10 is consumed, electric power is not stored in the secondary battery 10 again, so that the functions are stopped. Accordingly, the number of times the capsule medical apparatus 1 is used is limited to once.

Advantages of the case where the capsule medical apparatus 1 incorporates the secondary battery 10 are explained below. It is usually difficult to replace batteries of capsule medical apparatuses after they are shipped. For this reason, when adapting a primary battery that is not rechargeable, it is required to incorporate a primary battery that has electric power larger than that necessary for operations of a function executing unit in a capsule medical apparatus during assembling. In other words, it is required that the primary battery incorporated in the capsule medical apparatus has a large amount of electric power including not only electric power for operations of the function executing unit but also electric power that is naturally excreted during the period from the assembling of the apparatus until a user uses the apparatus, including the period in which the apparatus is stored before it is shipped. Therefore, it is required that the capsule medical apparatus incorporate an unnecessarily-large capsule medical apparatus or a large number of primary batteries, which makes it difficult to downsize the capsule medical apparatus and make full use of the performance of the incorporated battery.

In contrast, with the capsule medical apparatus 1 that incorporates the secondary battery 10 that is rechargeable, the secondary battery 10 can be charged at desired timing, for example, when the user uses the apparatus. Therefore, it is not necessary to store, in the secondary battery 10, electric power other than electric power for operations of the function executing unit 15, i.e., unnecessary electric power that is naturally excreted in, for example, the storage period. This makes it possible to downsize the capsule medical apparatus 1 and make full use of the performance of the secondary battery 10 which is incorporated. Incorporating the secondary battery 10 leads to advantages that downsizing the capsule medical apparatus 1 can be promoted, and that the electric power stored in the capsule medical apparatus 1 can be efficiently used for operations of the function executing unit 15.

As described above, in the first embodiment of the present invention, the receiving coil that converts an external magnetic field to electric power and the secondary battery that supplies electric power to the function executing unit are connected through the fuse, and the electric power input by the receiving coil is supplied to the secondary battery through the fuse to charge the secondary battery. After charging the secondary battery is complete, the fuse is disconnected, so that the connection between the receiving coil and the secondary cell is disconnected, which inhibits charging the secondary battery. Therefore, the number of times the incorporated secondary battery is used is limited to once, which assuredly inhibits the function executing unit from operating after the electric power, which is stored in the secondary battery by charging it only once, is consumed. Accordingly, can be achieved a capsule medical apparatus that has advantages obtained by incorporating the secondary battery, and that is inhibited from being reused unintentionally after being used for a subject, which limits the number the apparatus is used to only one, and a method of charging the capsule medical apparatus.

Modification 1 of the first embodiment of the present invention is explained below. In the first embodiment, the conduction path between the secondary battery 10 and the receiving coil 11 is a single path that does not branch off. In contrast, in Modification 1 of the first embodiment, a discharging path that branches off the conduction path between the secondary battery 10 and the receiving coil 11 is formed, and electric power used to disconnect the fuse 12b flows into the discharging path.

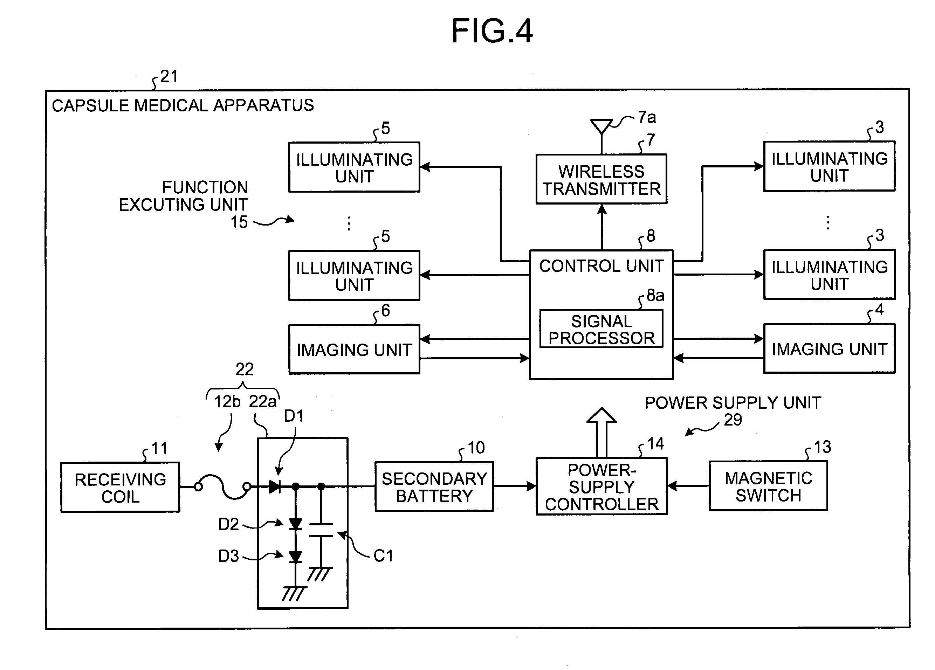

FIG. 4 is a schematic block diagram of a functional configuration example of a capsule medical apparatus according to Modification 1 of the first embodiment. As shown in FIG. 4, a capsule medical apparatus 21 according to Modification 1 of the first embodiment includes a power supply unit 29 instead of the power supply unit 9 of the capsule medical apparatus 1 according to the first embodiment. The power supply unit 29 includes a rectifier circuit 22a that has a discharging path for discharging electric power instead of the rectifier circuit 12a of the connecting circuit 12. Although is not shown in FIG. 4, the capsule medical apparatus 21 includes the capsule casing 2 identical with that (see FIG.1) of the capsule medical apparatus 1 according to the first embodiment. The configuration of the Modification 1 excluding the above aspects is the same as that of the first embodiment, and the identical elements are denoted by the same reference numerals.

The power supply unit 29 includes a connecting circuit 22 having the discharging path instead of the connecting circuit 12 of the capsule medical apparatus 1 according to the first embodiment. In addition to including the connecting circuit 22, the power supply unit 29 has the function identical with that of the power supply unit 9 of the capsule medical apparatus 1 according to the first embodiment.

The connecting circuit 22 includes the rectifier circuit 22a instead of the rectifier circuit 12a of the capsule medical apparatus 1 according to the first embodiment, and includes the fuse 12b. The connecting circuit 22 releasably connects the secondary battery 10 and the receiving coil 11, using the fuse 12b and the rectifier circuit 22a.

The rectifier circuit 22a includes the diode D1 and the capacitor C1, and further includes the diodes D2 and D3 that form the discharging path that branches off the conduction path, which is formed by the fuse 12b and the diode D1, between the secondary battery 10 and the receiving coil 11.

Specifically, an input terminal of the diode D2 is connected to an output terminal of the diode D1, and an output terminal of the diode D2 is connected to an input terminal of the diode D3. An output terminal of the diode D3 is connected to a ground potential. The electric power supplied from the receiving coil 11 to the secondary battery 10 is partly discharged to the discharging path formed by the diodes D2 and D3 in the period in which the secondary battery 10 is charged. A part of the electric power that flows into the discharging path does not inhibit charging the secondary battery 10. When charging the secondary battery 10 is complete, i.e., when disconnecting the fuse 12b to inhibit charging the secondary battery 10, a current that is generated in the receiving coil 11 due to the external magnetic field, and that is large enough to disconnect the fuse 12b, flows into the discharging path formed by the diodes D2 and D3. After the fuse 12 is disconnected, the overcurrent from the receiving coil 11 hardly flows into the conduction path on the side of the secondary battery and flows into the discharging path formed by the diodes D2 and D3. Thus, when causing the overcurrent to flow into the conduction path between the secondary battery 10 and the receiving coil 11 to disconnect the fuse 12b, the load of the secondary battery 10 having been charged can be reduced, and the excessive current sufficient to disconnect the fuse 12b can be easily input to the fuse 12b.

It is desirable that the sum of forward voltages of the diodes D2 and D3 that form the discharging path be equal to or higher than an open circuit voltage of the secondary battery 10, which prevents the electric power in the secondary battery 10 from flowing toward the discharging path formed by the diodes D2 and D3. The number of diodes that form the discharging path is not limited to two, and it suffices that at least one diode is used. It is also desirable that the sum of forward voltage of at least one diode be equal to or higher than the open circuit voltage of the secondary battery 10.

As explained above, in Modification 1 according to the first embodiment of the present invention, the discharging path that branches off the conduction path between the secondary battery and the receiving coil is formed by at least one diode. When inhibiting charging the secondary battery, an overcurrent sufficient to disconnect the fuse in the conduction path is caused to flow into the discharging path through the fuse. The configuration of Modification 1 of the first embodiment excluding the above aspects is the same as that of the first embodiment. Because of the configuration, a current that flows toward the secondary battery having been charged can be reduced, and an overcurrent sufficient to disconnect the fuse can be caused to flow into the fuse easily. Accordingly, the same functions and effects as those of the first embodiment can be achieved. In addition, after charging the secondary battery is complete, while the load of the secondary battery having been charged is reduced, the fuse in the conduction path is assuredly disconnected to assuredly inhibit charging the secondary battery.

Modification 2 of the first embodiment of the present invention is explained below. In the first embodiment, the fuse 12b is disconnected with an overcurrent generated in the receiving coil 11 due to an external magnetic field. In contrast, in Modification 2 of the first embodiment, the fuse 12b is disconnected using a part of the electric power stored in the secondary battery 10.

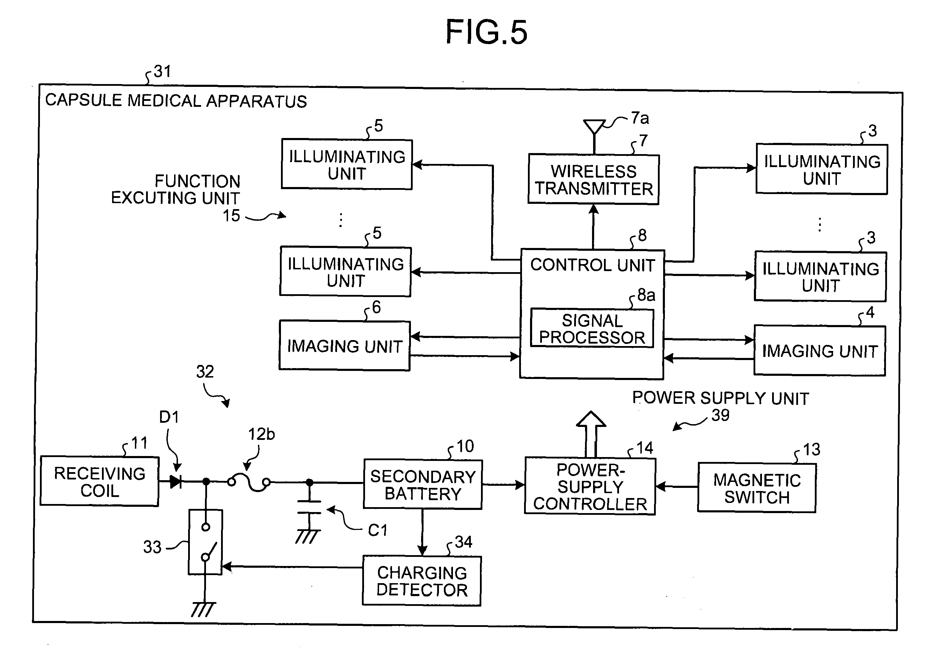

FIG. 5 is a schematic block diagram of a functional configuration example of a capsule medical apparatus according to Modification 2 of the first embodiment of the present invention. As shown in FIG. 5, a capsule medical apparatus 31 according to Modification 2 of the first embodiment includes a power supply unit 39 instead of the power supply unit 9 of the capsule medical apparatus 1 according to the first embodiment. The power supply unit 39 includes a connecting circuit 32 instead of the connecting circuit 12, and further includes a charging detector 34 that detects that charging the secondary battery 10 is complete. The fuse 12b is arranged between the diode D1 and the capacitor C1. Although it is not shown in FIG. 5, the capsule medical apparatus 31 includes the capsule casing 20 identical with that (see FIG. 1) of the capsule medical apparatus 1 according to the first embodiment. The configuration of Modification 2 excluding the above aspects is the same as that of the first embodiment, and the identical elements are denoted by the same reference numerals.

The power supply unit 39 detects that charging the secondary battery 10 is complete using the charging detector 34. When charging the secondary battery 10 is complete, power supply unit 39 disconnects the fuse 12b using a part of the electric power in the secondary battery 10 having been charged, thereby inhibiting charging the secondary battery 10. The power supply unit 39 has the same functions as those of the power supply unit 9 of the capsule medical apparatus 1 according to the first embodiment, excluding the above function of inhibiting charging the secondary battery 10.

The connecting circuit 32 includes the fuse 12b, the diode D1, and the capacitor C1, and releasably connects the secondary battery 10 and the receiving coil 11 using the fuse 12b and the diode D1. The connecting circuit 32 includes a switching unit 33 that forms a discharging path that branches off a conduction path between the secondary battery 10 and the receiving coil 11. In the connecting circuit 32, the fuse 12b and the diode D1 form the conduction path between the secondary battery 10 and the receiving coil 11, and the diode D1 and the capacitor C1 constitute a rectifier circuit of the conduction path between the secondary battery 10 and the receiving coil 11 like the rectifier circuit 12a. One terminal of the fuse 12b is connected to an output terminal of the diode D1 and the switching unit 33, and the other terminal of the fuse 12b is connected to the capacitor C1 and the secondary battery 10.

One terminal of the switching unit 33 is connected to the output terminal of the diode D1 and the fuse 12b, and the other terminal of the switching unit 33 is connected to a ground potential, thereby forming the discharging path that branches off the conduction path between the secondary battery 10 and the receiving coil 11. At an initial state before charging the secondary battery 10 is complete, the switching unit 33 is open and the discharging path is blocked. In contrast, when charging the secondary battery 10 is complete, the switching unit 33 is closed under the control of the charging detector 34, thereby unblocking the discharging path. A part of the electric power from the secondary battery 10 is discharged to the discharging path, which is achieved while the switching unit 33 is closed, through the fuse 12b.

The charging detector 34 monitors the current or voltage of the electric power that is supplied from the receiving coil 11 to the secondary battery 10 through the connecting circuit 32. When the current is below a predetermined threshold or the voltage is equal to or over a predetermined threshold, the charging detector 34 detects that charging the secondary battery 10 is complete. When charging the secondary battery 10 is not complete, the charging detector 34 controls the switching unit 33 such that it enters the open state to block the discharging path achieved by the switching unit 33. When charging the secondary battery 10 is complete, the charging detector 34 controls the switching unit 33 such that it is closed to electrically connect the discharging path, which is achieved by the switching unit 33 and the secondary battery 10 through the fuse 12b.

When the discharging path achieved by the switching unit 33 and the secondary battery 10 are electrically connected through the fuse 12b, a part of the electric power stored in the secondary battery 10 flows as a current into the fuse 12b rapidly and is discharged to the discharging path achieved by the switching unit 33 through the fuse 12b. In this case, the fuse 12b is disconnected due to the action of the rapid current from the secondary battery 10, thereby inhibiting charging the secondary battery 10. At the same time, the switching unit 33 and the secondary battery 10 are not electrically connected due to disconnection of the fuse 12b, so that discharging to the fuse 12b by the secondary battery 10 is stopped. The switching unit 33 may maintain the closed state after the fuse 12b is disconnected, or returns to the open state under the control of the charging detector 34. Furthermore, a resistor (not shown) that limits currents discharged from the secondary battery 10 may be provided in the path from the secondary battery 10 to the ground potential through the fuse 12b and the switching unit 33.

As explained above, in Modification 2 of the first embodiment of the present invention, the switching unit that can be open or closed forms the discharging path that branches off the conduction path between the secondary battery and the receiving coil. The charging detector detects that charging the secondary battery is complete. When charging the secondary battery is complete, the charging detector opens or closes the switching unit to allow a rapid current from the secondary battery having been charged flow into the discharging path through the fuse in the conduction path. The configuration of Modification 2 of the first embodiment excluding the above aspects is the same as that of the first embodiment. Because of the configuration, when disconnecting the fuse in the conduction path between the secondary battery and the receiving coil to inhibit charging the secondary battery, the fuse in the conduction path can be disconnected due to the rapid current from the secondary battery having been charged without input of an excessive current generated by the receiving coil from an external magnetic field to the fuse. Accordingly, the same functions and effects as those of the first embodiment can be achieved. In addition, after charging the secondary battery is complete, the fuse in the conduction path can be easily disconnected to easily inhibit charging the secondary battery easily, while the load of the secondary battery having been discharged is reduced.

A second embodiment of the present invention is explained below. In the first embodiment, charging the secondary battery 10 is inhibited by disconnecting the fuse 12b that serves as a part of the conduction path between the secondary battery 10 and the receiving coil 11. In contrast, in the second embodiment, a semiconductor switching device is arranged in the conduction path between the secondary battery 10 and the receiving coil 11, and charging the secondary battery 10 is inhibited by switching the semiconductor switching device to the open state.

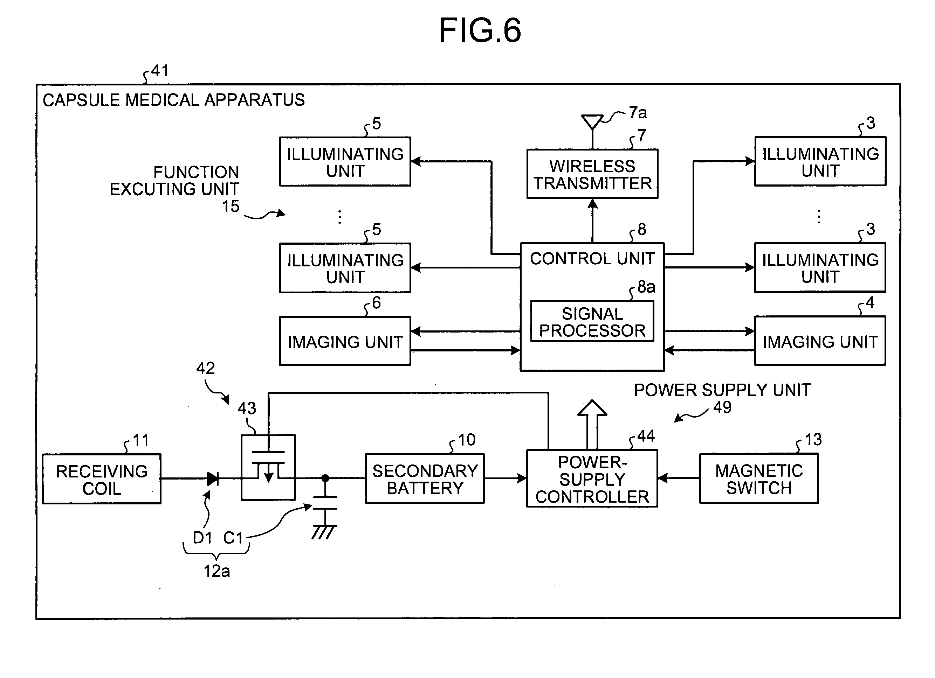

FIG. 6 is a schematic block diagram of a functional configuration example of a capsule medical apparatus according to the second embodiment of the present invention. As shown in FIG. 6, a capsule medical apparatus 41 according to the second embodiment includes a power supply unit 49 instead of the power supply unit 9 of the capsule medical apparatus 1 according to the first embodiment. The power supply unit 49 includes a connecting circuit 42 instead of the connecting circuit 12, and includes a power-supply controller 44 instead of the power-supply controller 14. Although it is not shown in FIG. 6, the capsule medical apparatus 41 includes the capsule casing 2 identical with that (see FIG. 1) of the capsule medical apparatus 1 according to the first embodiment. The configuration of the second embodiment excluding the above aspects is the same as that of the first embodiment, and the identical elements are denoted by the same reference numerals.

In the OFF state where the electric power from the secondary battery 10 is not supplied to the function executing unit 15, the power supply unit 49 connects the secondary battery 10 and the receiving coil 11 through the connecting circuit 42. In the ON state where the electric power from the secondary battery 10 is supplied to the function executing unit 15, the power supply unit 49 releases the connection between the secondary battery 10 and the receiving coil 11 through the connecting circuit 42, thereby inhibiting charging the secondary battery 10. The power supply unit 49 has the same function as those of the power supply unit 9 of the capsule medical apparatus 1 according to the first embodiment, excluding the function of switching between the state where the secondary battery 10 and the receiving coil 11 are electrically connected through the connecting circuit 42 and the state where they are not electrically connected through the connecting circuit 42.

The connecting circuit 42 releasably connects the secondary battery 10 and the receiving coil 11 by switching the conductive/non-conductive state of the conduction path between the secondary battery 10 and the receiving coil 11. The connecting circuit 42 includes the diode D1, the capacitor C1, and a semiconductor switching device 43 that switches the conductive/non-conductive state of the conduction path between the secondary battery 10 and the receiving coil 11. The diode D1 and the capacitor C1 constitutes the rectifier circuit 12a of the conduction path between the secondary battery 10 and the receiving coil 11, which is formed by the connecting circuit 42.

The semiconductor switching device 43 releasably connects the secondary battery 10 and the receiving coil 11. Specifically, the semiconductor switching device 43 is a field-effect transistor, and it is arranged between the diode D1 and the capacitor C1 as shown in FIG. 6. The semiconductor switching device 43 forms a part of the conduction path between the secondary battery 10 and the receiving coil 11, i.e., the parts of the conduction path excluding the part formed by the rectifier circuit 12a.
The semiconductor switching device 43 opens or is closed under the control of the power-supply controller 44 to switch the conduction path to the conductive state or the non-conductive state. More specifically, before the electric power from the secondary battery 10 is supplied to the function executing unit 15, i.e., during the OFF state, the semiconductor switching device 43 maintains the closed state under the control of the power-supply controller 44 to maintain the state where the secondary battery 10 and the receiving coil 11 are connected to each other. In contrast, after supplying the eclectic power from the secondary battery 10 to the function executing unit 15 is started, i.e., during the ON state, the semiconductor switching device 43 is switched to the open state under the control of the power-supply controller 44, thereby releasing the connection between the secondary battery 10 and the receiving coil 11. Thereafter, the semiconductor switching device 43 maintains the open state to continue releasing the connection between the secondary battery 10 and the receiving coil 11, thereby inhibiting charging the secondary battery 10.

The power-supply controller 44 has a function of controlling ON/OFF of the power supply unit 49 as the power-supply controller 14 according to the fist embodiment has. The power-supply controller 44 also functions as a connection control unit that controls releasing of the connection between the secondary battery 10 and the receiving coil 11 through the connecting circuit 42. Specifically, the power-supply controller 44 maintains the OFF state of the power supply unit 49 in the initial state before an instruction signal for starting supplying electric power is input from the magnetic switch 13, and controls the semiconductor switching device 43 such that it enters the closed state, thereby maintaining the conductive state of the conduction path between the secondary battery 10 and the receiving coil 11. When the power-supply controller 44 receives the instruction signal for starting supplying electric power from the magnetic switch 13, the power-supply controller 44 switches the power supply unit 49 ON to start supplying the electric power from the secondary battery 10 to the function executing unit 15, and controls the semiconductor switching device 43 such that it enters the open state, thereby switching the conduction path between the secondary battery 10 and the receiving coil 11 to the non-conductive state as in the case of the first embodiment. In this manner, the power-supply controller 44 releases the connection between the secondary battery 10 and the receiving coil 11. Thereafter, the power-supply controller 44 maintains the ON state of the power supply unit 49 and maintains the open state of the semiconductor switching device 43 to continue releasing the connection between the secondary battery 10 and the receiving coil 11, thereby inhibiting charging the secondary battery 10. The power-supply controller 44 continues releasing the connection between the secondary battery 10 and the receiving coil 11 to inhibit charging the secondary battery 10, even after the electric power in the secondary battery 10 is consumed.

When the power-supply controller 44 receives the instruction signal for stopping supplying electric power from the magnetic switch 13, after starting supplying the electric power from the secondary battery 10 to the function executing unit 15, the power-supply controller 44 may stop supplying the electric power from the secondary battery 10 to the function executing unit 15 based on the received instruction signal. Even after stopping to supply the electric power from the secondary battery 10, the power-supply controller 44 maintains the open state of the semiconductor switching device 43 to continue releasing the connection between the secondary battery 10 and the receiving coil 11, thereby inhibiting charging the secondary battery 10.

As explained above, in the second embodiment of the present invention, the receiving coil that converts an external magnetic field to electric power and the secondary battery that supplies electric power to the function executing unit are connected to each other through the semiconductor switching device. The electric power input by the receiving coil is supplied to the secondary battery through the semiconductor switching device to charge the secondary battery. After supplying the electric power from the secondary battery to the function executing unit is started, the semiconductor switching device is switched to the open state (non-conductive state) to release the connection between the receiving coil and the secondary battery, thereby inhibiting charging the secondary battery. The configuration of the second embodiment excluding the above aspects is the same as that of the first embodiment. Because of the configuration, the number of times the secondary battery can be charged is limited to once as in the case of the first embodiment, and the electric power necessary for releasing the connection between the receiving coil and the secondary battery can be reduced. Accordingly, the same functions and effects as those of the first embodiment can be achieved, and the electric power necessary for inhibiting charging the secondary battery can be reduced.

Modification 1 of the second embodiment of the present invention is explained below. In the second embodiment, the connection between the secondary battery 10 and the receiving coil 11 is released when the power supply unit 49 is switched ON from OFF. In contrast, in Modification 1 of the second embodiment, the apparatus further includes a detector that detects predetermined external information, and the connection between the secondary battery 10 and the receiving coil 11 is released when the detector detects external information.

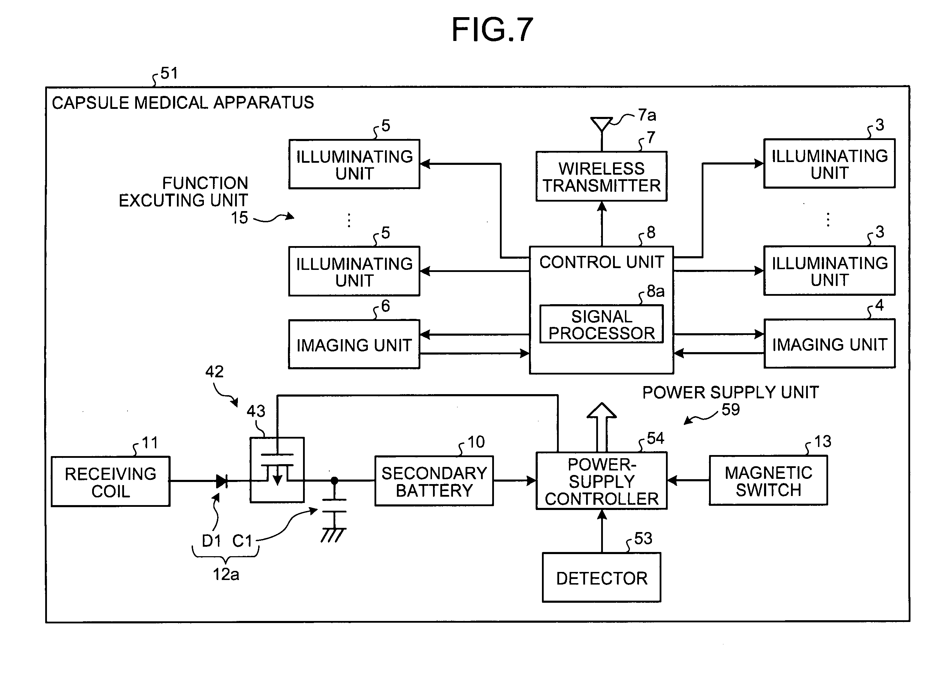

FIG. 7 is a schematic block diagram of a functional configuration example of a capsule medical apparatus according to Modification 1 of the second embodiment of the present invention. As shown in FIG. 7, a capsule medical apparatus 51 according to the modification 1 of the second embodiment includes a power supply unit 59 instead of the power supply unit 49 of the capsule medical apparatus 41 according to the second embodiment. The power supply unit 59 includes a power-supply controller 54 instead of the power-supply controller 44, and further includes a detector 53 that detects predetermined external information. Although it is not shown in FIG. 7, the capsule medical apparatus 51 includes the capsule casing 2 identical with that (see FIG. 1) of the capsule medical apparatus 1 according to the first embodiment. The configuration of Modification 1 of the second embodiment excluding the above aspects is the same as that of the second embodiment, and identical elements are denoted by the same reference numerals.

The power supply unit 59 releases the connection between the secondary battery 10 and the receiving coil 11 through the connecting circuit 42 based on a result of detecting external information, such as information about magnetic field or light outside of the capsule medical apparatus, instead of the magnetic signal of the predetermined pattern applied to switch the ON/OFF state, thereby inhibiting charging the secondary battery 10. The power supply unit 59 has the same functions as those of the power supply unit 49 of the capsule medical apparatus 41 according to the second embodiment, excluding the function of releasing the connection between the secondary battery 10 and the receiving coil 11.

The detector 53 functions as an information detecting unit that detects external information, such as information about magnetic field or light outside the capsule medical apparatus. The detector 53 monitors whether there is the predetermined external information. When there is the predetermined external information outside the capsule medical apparatus 51, the detector 53 detects the external information and sends a detection signal representing the result of detecting the external information to the power-supply controller 54.

The external information detected by the detector 53 is, for example, information in a predetermined pattern about any one of magnetic field, light, high frequency, ultrasound, temperature, and pH outside the capsule medical apparatus 51. The information about magnetic field, light, high frequency, and ultrasound may be applied to the capsule medical apparatus 51 by a predetermined external device, and the information about temperature and pH information may be the temperature or pH that is detectable by the capsule medical apparatus 51 inside the subject.

The power-supply controller 54 controls releasing the connection between the secondary battery 10 and the receiving coil 11 through the connecting circuit 42 based on the result of detecting the external information by the detector 53. Specifically, before the detector 53 detects the predetermined external information, the power-supply controller 54 controls the semiconductor switching device 43 such that it is closed to maintain the conductive state of the conduction path between the secondary battery 10 and the receiving coil 11. In contrast, when the power-supply controller 54 receives the detection signal representing that the external information is detected from the detector 53, the power-supply controller 54 controls the semiconductor switching device 43 such that it enters the open state to switch the conduction path between the secondary battery 10 and the receiving coil 11 to the non-conductive state, thereby releasing the connection between the secondary battery 10 and the receiving coil 11. Thereafter, the power-supply controller 54 maintains the open state of the semiconductor switching device 43 to continue releasing the connection between the secondary battery 10 and the receiving coil 11, thereby inhibiting charging the secondary battery 10. Other functions of the power-supply controller 54 are the same as those of the power-supply controller 44 of the capsule medical apparatus 41 according to the second embodiment.

After charging the secondary battery 10 is complete, the information in the predetermined pattern about any one of magnetic field, light, high frequency, and ultrasound, which is external information, is applied to the capsule medical apparatus 51 having the above configuration at timing that the user desires, for example, before the capsule medical apparatus 51 is introduced into the subject. The detector 53 detects the applied external information. The power-supply controller 54 controls the semiconductor switching device 43 such that it enters the open state based on the result of detecting the external information by the detector 53 to release the connection between the secondary battery 10 and the receiving coil 11. Thereafter, the capsule medical apparatus 51 maintains the state where charging the secondary battery 10 is inhibited.

Alternatively, the capsule medical apparatus 51 is introduced into the subject after charging the secondary battery 10 is complete, and the detector 53 detects the temperature inside the subject or pH as the external information outside the capsule medical apparatus 51. The power-supply controller 54 controls the semiconductor switching device 43 such that it enters the open state based on the result of detecting the information about temperature or pH in the subject to release the connection between the secondary battery 10 and the receiving coil 11. Thereafter, the capsule medical apparatus 51 maintains the state where charging the secondary battery is inhibited.

As explained above, in Modification 1 of the second embodiment of the present invention, the detector detects the predetermined external information different from the external magnetic field for switching ON/OFF the power supply unit. Based on the result of detecting the external information, the semiconductor switching device in the conduction path between the secondary battery and the receiving coil is switched to the open state, i.e., non-conductive state. This releases the connection between the receiving coil and the secondary battery, thereby inhibiting charging the secondary battery. The configuration of Modification 1 of the second embodiment excluding the above aspects is the same as that of the second embodiment. Because of the configuration, the same functions and effects as those of the second embodiment can be achieved, and charging the secondary battery can be inhibited not at the timing at which ON/OFF of the power supply unit is switched but at desired timing after charging the secondary battery is complete.

Modification 2 of the second embodiment of the present invention is explained below. In the second embodiment, the connection between the secondary battery 10 and the receiving coil 11 is released when the power supply unit 49 is switched to ON from OFF. In contrast, in Modification 2 of the second embodiment, the connection between the secondary battery 10 and the receiving coil 11 is released when charging the secondary battery 10 is competed.

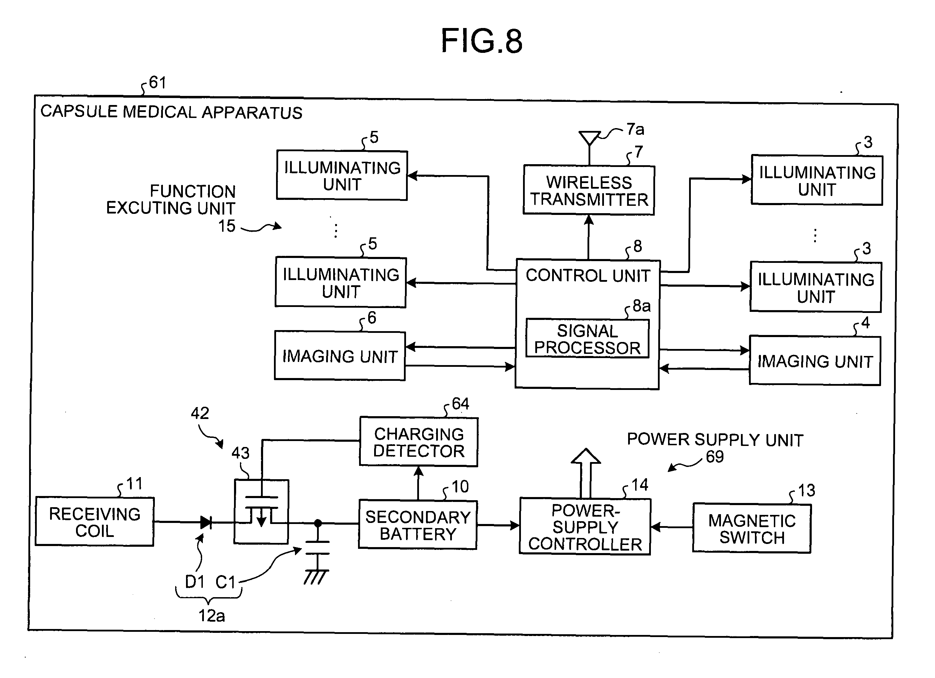

FIG. 8 is a schematic block diagram of a functional configuration example of a capsule medical apparatus according to Modification 2 of the second embodiment. As shown in FIG. 8, a capsule medical apparatus 61 according to Modification 2 of the second embodiment includes a power supply unit 69 instead of the power supply unit 49 of the capsule medical apparatus 41 according to the second embodiment. The power supply unit 69 includes, instead of the power-supply controller 44, the power-supply controller 14 identical with that of the capsule medical apparatus 1 according to the first embodiment, and further includes a charging detector 64 that detects that charging the secondary battery 10 is complete. Although it is not shown in FIG. 8, the capsule medical apparatus 61 includes the capsule casing 2 (see FIG. 1) identical with that of the capsule medical apparatus 1 according to the first embodiment. The configuration of Modification 2 of the second embodiment excluding the above aspects is the same as that of the second embodiment, and identical elements are denoted by the same reference numerals.

The power supply unit 69 releases the connection between the secondary battery 10 and the receiving coil 11 through the connecting circuit 42 at the timing at which charging the secondary battery 10 is complete, thereby inhibiting charging the secondary battery 10. The power supply unit 69 has the same functions as those of the power supply unit 49 of the capsule medical apparatus 41 according to the second embodiment, excluding the above function of releasing the connection between the secondary battery 10 and the receiving coil 11.

The charging detector 64 monitors the current or voltage of the electric power that is supplied from the receiving coil 11 to the secondary battery 10 through the connecting circuit 42. When the current is below a predetermined threshold or the voltage is equal to or over a predetermined threshold, the charging detector 64 detects that charging the secondary battery 10 is complete. When charging the secondary battery 10 is not complete, the charging detector 64 controls the semiconductor switching device 43 such that it is closed to maintains the conductive state of the conduction path between the secondary battery and the receiving coil 11. In contrast, When charging the secondary battery 10 is complete, the charging detector 64 controls the semiconductor switching device 43 such that it enters the open state to switch the conduction path between the secondary battery 10 and the receiving coil 11 to the conductive state, thereby releasing the connection between the secondary battery 10 and the receiving coil 11. Thereafter, the charging detector 64 maintains the open state of the semiconductor switching device 43 to continue releasing the connection between the secondary battery 10 and the receiving coil 11, thereby inhibiting charging the secondary battery 10.

As explained above, in Modification 2 of the second embodiment of the present invention, the charging detector detects that charging the secondary battery is complete. When charging the secondary battery is complete, the semiconductor switching device in the conduction path between the receiving coil and the secondary battery is switched to the open state, i.e., the non-conductive state. This releases the connection between the receiving coil and the secondary battery, which inhibits charging the secondary battery. The configuration of Modification 2 of the second embodiment excluding the above aspects is the same as that of the second embodiment. Because of the configuration, the same functions and effects as those of the second embodiment can be achieved, and charging the secondary battery can be assuredly inhibited after charging the secondary battery is complete.

A third embodiment of the present invention is explained below. In the first and second embodiments and each of Modifications, the number of times a capsule medical apparatus is used is limited to once by inhibiting inhibition charging the secondary battery 10. In contrast, in the third embodiment, after supplying the electric power from the secondary battery 10 to the function executing unit 15 is started, supplying electric power to the function executing unit 15 is stopped at predetermined timing. Thereafter, the state where supplying electric power is stopped is maintained. This limits the number of times the capsule medical apparatus is used to one.

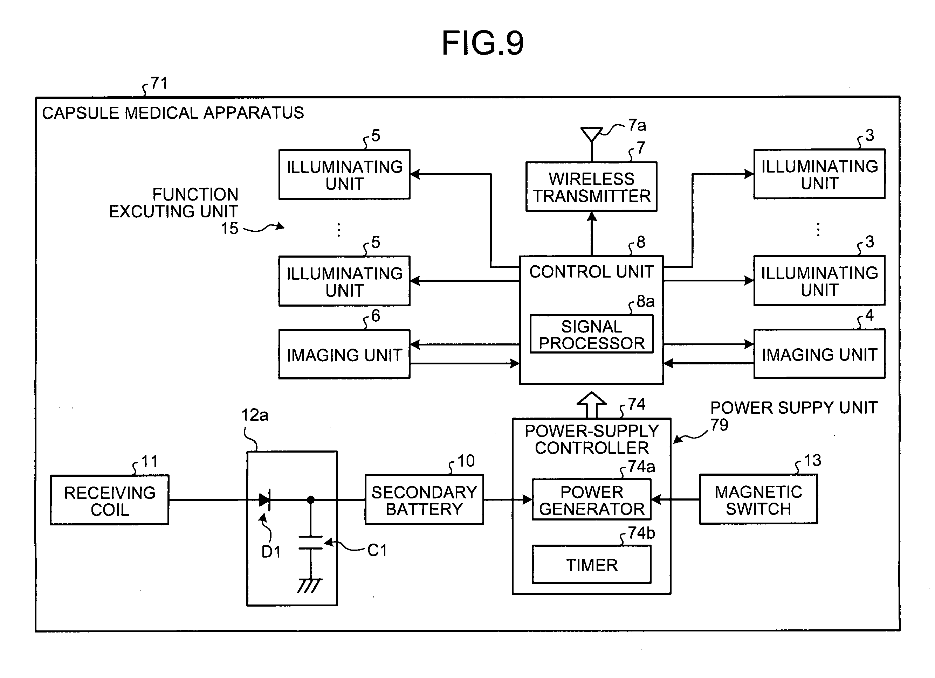

FIG. 9 is a schematic block diagram of a configuration example of a capsule medical apparatus according to the third embodiment of the present invention. As shown in FIG. 9, a capsule medical apparatus 71 according to the third embodiment includes a power supply unit 79 instead of the power supply unit 9 of the capsule medical apparatus 1 according to the first embodiment. The power supply unit 79 includes a power-supply controller 74 instead of the power-supply controller 14. The power supply unit 79 does not include the fuse 12b, and the conduction path between the secondary battery 10 and the receiving coil 11 is formed by the rectifier circuit 12a. Although it is not shown in FIG. 9, the capsule medical apparatus 71 includes the capsule casing 2 (see FIG. 1) identical with that of the capsule medical apparatus 1 according to the first embodiment. The configuration of the third embodiment excluding the above aspects is the same as that of the first embodiment, and identical elements are denoted by the same reference numerals.

The power supply unit 79 has the function of supplying electric power to the function executing unit 15 as the power supply unit 9 of the capsule medical apparatus 1 according to the first embodiment has. In addition, instead of the function of inhibiting charging the secondary battery, the power supply unit 79 has a function of stopping to supply electric power to the function executing unit 15 at predetermined timing after supplying electric power to the function executing unit 15 is started.

The power-supply controller 74 has the function of controlling ON and OFF of the power supply unit 79 based on instruction information from the magnetic switch 13, as the power-supply controller 14 according to the first embodiment has. In addition, the power-supply controller 74 has a function of controlling the operation period of the function executing unit 15 by stopping to supply electric power to the function executing unit 15 at timing when a predetermined time elapses from when supplying the electric power from the secondary battery 10 to the function executing unit 15 is started.

Specifically, the power-supply controller 74 includes a power generator 74a that generates electric power to be supplied to the function executing unit 15, and a timer 74b that measures a time that elapses from when supplying electric power to the function executing unit 15 is started. The power generator 74a generates operation power for the function executing unit 15 based on the electric power in the secondary battery 10. The power-supply controller 74 starts supplying the generated operation power to the function executing unit 15 based on an instruction signal for starting supplying electric power from the magnetic switch 13. The timer 74b starts a counting process for time information at timing when the timer 74b receives instruction information for starting supply electric power from the magnetic switch 13, i.e., the timing where supplying the operation power to the function executing unit 15 is started. Specifically, the timer 74b successively counts pulses from, for example, a crystal oscillator to measure the elapsed time from when supplying the operation power to the function executing unit 15 is started. When the counted value that is the time information obtained through the counting process of the timer 74b, i.e., the elapsed time from when supplying the operation power to the function executing unit 15 is started, reaches a predetermined value, the power-supply controller 74 stops supplying the operation power to the function executing unit 15, thereby stopping operations of the function executing unit 15.

When the power-supply controller 74 receives the instruction signal for stopping to supply electric power from the magnetic switch 13 after starting supplying the electric power from the secondary battery 10 to the function executing unit 15, the power-supply controller 74 may temporarily stop supplying the electric power to the function executing unit 15 based on the instruction information for stopping supplying electric power, or maintain the state where supplying the operation power is stopped not depending on a result of the counting process of the timer 74b. When the power-supply controller 74 temporarily stops supplying the operation power based on the instruction information for stopping supplying electric power, the power-supply controller 74 may temporarily stop the counting process of the timer 74b as well, or continue performing the counting process of the timer 74b not depending on the state where supplying the operation power is temporarily stopped.

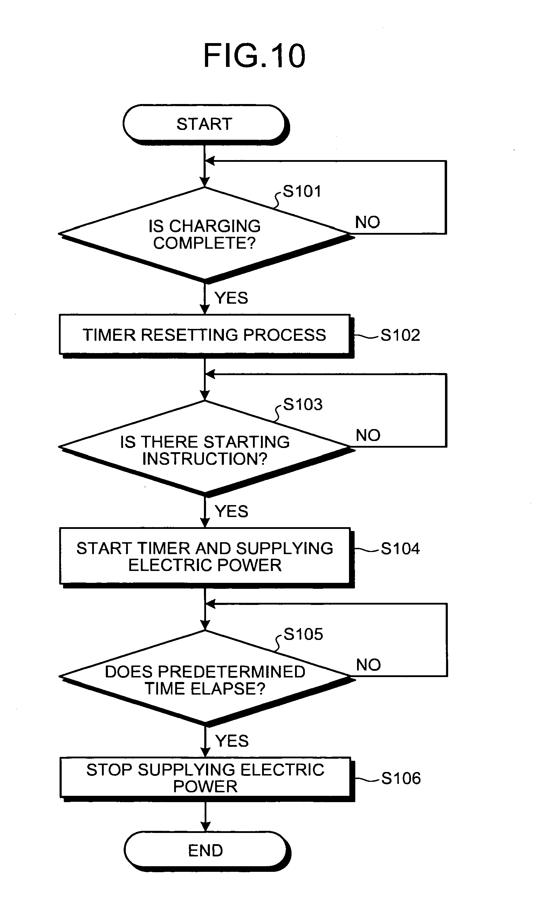

Subsequently, a procedure performed by the power-supply controller 74 for controlling supplying electric power from the secondary battery 10 to the function executing unit 15 of the capsule medical apparatus 71 to control stopping and starting operations of the function executing unit 15. FIG. 10 is a flowchart of an example of the procedure performed by the power-supply controller 74 of the capsule medical apparatus 71 according to the third embodiment.

As shown in FIG. 10, the power-supply controller 74 determines whether charging the secondary battery 10 is complete (step S101). At step S101, the power-supply controller 74 acquires a value of the current or voltage of electric power supplied from the receiving coil 11 to the secondary battery 10 through the rectifier circuit 12a. When the current is below a predetermined threshold, or when the voltage is equal to or above a predetermined threshold, the power-supply controller 74 determines that charging the secondary battery 10 is complete.

When charging the secondary battery 10 is not complete (NO at step S101), the power-supply controller 74 repeats the process at step S101 until charging the secondary battery 10 is complete. In contrast, when charging the secondary battery 10 is complete (YES at step S101), the power-supply controller 74 performs a resetting process for initializing the counted value in the counting process of the timer 74b to, for example, zero (step S102), and determines whether there is an instruction for starting the function executing unit 15 (step S103).

At step S103, when the power-supply controller 74 does not receive the instruction information for starting supplying power from the magnetic switch 13, the power-supply controller 74 determines that there is no instruction for starting the function executing unit 15 (NO at step S103), and repeats the process at step S103. In other words, the power-supply controller 74 waits until the magnetic switch 13 inputs instruction information for starting power supply. In contrast, when the power-supply controller 74 receives instruction information for starting supplying electric power from the magnetic switch 13, the power-supply controller 74 determines that there is an instruction for starting the function executing unit 15 (YES at step S103), the power-supply controller 74 starts the timer 74b and starts supplying the electric power from the secondary battery 10 to the function executing unit 15 (step S104).

At step S104, the power generator 74a generates operation power for the function executing unit 15 based on the electric power stored in the secondary battery 10, and starts supplying the generated operation power to the function executing unit 15. In this manner, the power-supply controller 74 causes the function executing unit 15 to start to operate. The timer 74b starts the counting process for time information at the same time when supplying the operation power to the function executing unit 15 is started, and obtains information about the elapsed time from when supplying the operation power is started, i.e., information about the operation time of the function executing unit 15.

Thereafter, the power-supply controller 74 determines whether a predetermined time elapses from when supplying the operation power to the function executing unit 15 is started (step S105). When the predetermined time does not elapse after supplying the operation power (NO at step S105), the power-supply controller 74 repeats the process at step S105. In this case, the timer 74b continues counting the elapsed time from when supplying the operation power is started, and the power generator 74a continues performing the process for generating operation power for the function executing unit 15. The power-supply controller 74 continues supplying the operation power to the function executing unit 15 to cause the function executing unit 15 to continue its operations.

In contrast, when the predetermined time elapses after starting supplying the operation power to the function executing unit 15 (YES at step S105), the power-supply controller 74 stops supplying the operation power to the function executing unit 15 (step S106) and completes the process. At steps S105 and S106, the timer 74b continues counting the elapsed time from when supplying electric power of the operation power is started. The power-supply controller 74 monitors the result of the counting process of the timer 74b. When the result of the counting process reaches a predetermined value, i.e., when the predetermined set time elapses from when supplying the operation power is started, the power-supply controller 74 stops supplying the operation power to the function executing unit 15. In this case, the power generator 74a stops the process for generating operation power for the function executing unit 15. In this manner, the power-supply controller 74 stops operations of the function executing unit 15. Thereafter, the power-supply controller 74 maintains the state where supplying the operation power to the function executing unit 15 is stopped to maintain the state where operations of the function executing unit 15 are stopped even when electric power remains in the secondary battery 10.

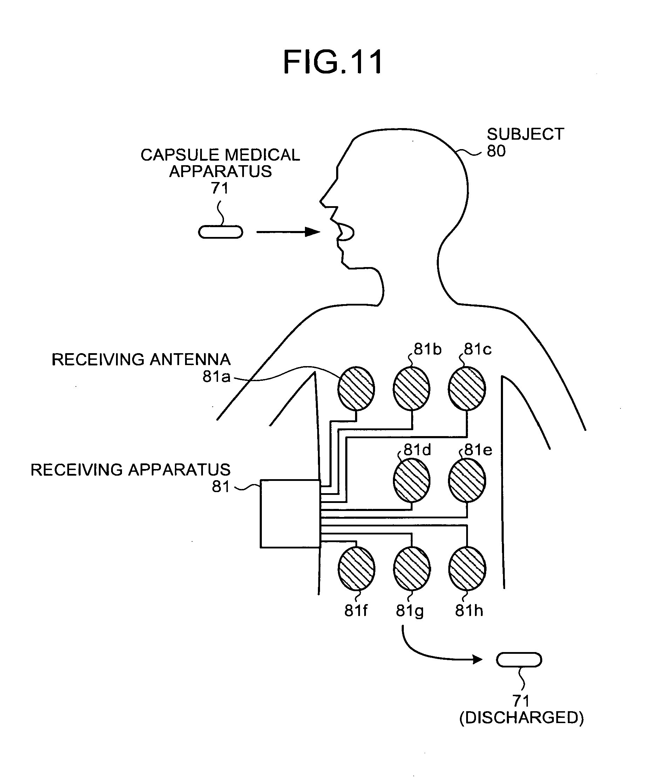

Operations of the capsule medical apparatus 71 before its operation is stopped when a predetermined time elapses after it is started are explained in detail below, taking the case where the capsule medical apparatus 71 is introduced into a subject from the mouth after it is booted. FIG. 11 is a schematic diagram representing how the capsule medical apparatus according to the third embodiment of the present invention is introduced into a subject from the mouth and excreted by the subject.

As shown in FIG. 11, the capsule medical apparatus 71 is started in response to a magnetic signal in a predetermined pattern from a predetermined external device (not shown) and then introduced into a subject 80 from the mouth. The capsule medical apparatus 71 in the subject 80 moves through the alimentary canal by peristalsis. In the capsule medical apparatus 71, the power generator 74a shown in FIG. 9 generates operation power for the function executing unit 15 based on the electric power in the secondary battery 10, and continues supplying the operation power to the function executing unit 15. The timer 74b continues counting the elapsed time from when supplying the operation power is started. The function executing unit 15 sequentially performs predetermined functions by consuming the operation power generated by the power generator 74a. Specifically, the function executing unit 15 takes in-vivo images of the subject 80 and wirelessly transmits signals containing the in-vivo images, sequentially.

In contrast, a receiving device 81 is carried by the subject 80 into which the capsule medical apparatus 71 is introduced from the mouth, and receiving antennas 81a to 81h are separately arranged on the body surface of the subject 80. The receiving device 81 sequentially receives wireless signals from the capsule medical apparatus 71, and performs a predetermined decoding process on the received wireless signals to extract the signals of the in-vivo images. The receiving device 81 generates in-vivo images of the subject 80 based on the signals of the in-vivo images. A portable recording medium (not shown) is detachably attached to the receiving device 81, and the receiving device 81 stores the in-vivo images of the subject 80 in the recording medium. After the capsule medical apparatus 71 is excreted by the subject 80, the recording medium in the receiving device 81 is attached to an image display device (not shown) that displays the in-vivo images of the subject 80. The number of receiving antennas of the receiving device 81 is not limited to eight. It suffices that at least one receiving antenna is used.

The capsule medical apparatus 71 in the subject 80 moves forward in the alimentary canal by, for example, peristalsis and is naturally excreted by the subject 80. At this point, a predetermined time has elapsed from when the capsule medical apparatus 71 is started, and the result of the counting process of the timer 74b in the capsule medical apparatus 71, i.e., the time information, reaches the predetermined value. In the capsule medical apparatus 71, the power generator 74a stops generating operation power for the function executing unit 15, thereby stopping operations of the function executing unit 15. Thereafter, the capsule medical apparatus 71 having been excreted, i.e., having been used, maintains the state where operations of the function executing unit 15 are stopped even when electric power remains in the secondary battery 10. Accordingly, the number of times the capsule medical apparatus 71 is used can be limited to once.

As explained above, in the third embodiment of the present invention, predetermined information successive from when operations of the function executing unit are started, such as time information, is obtained through the counting process. Instead of inhibiting charging the secondary battery, supplying the operation power to the function executing unit is stopped to stop operations of the function executing unit at the timing when the result of the counting process for the predetermined information reaches the predetermined set value. Thereafter, the state where operations of the function executing unit are stopped is maintained. The configuration of the third embodiment excluding the above aspects is the same as that of the first embodiment. Because of the configuration, advantages obtained by incorporating the secondary battery can be obtained as in the case of the first embodiment. In addition, the period in which the function executing unit can operate can be assuredly limited to the period from when the function executing unit is started, i.e., supplying the operation power is started, to when the predetermined time elapses. Accordingly, a capsule medical apparatus can be achieved that is not unintentionally reused even when operation power for the function executing unit remains in the power supply unit, which limits the number of times the capsule medical apparatus is used to once.

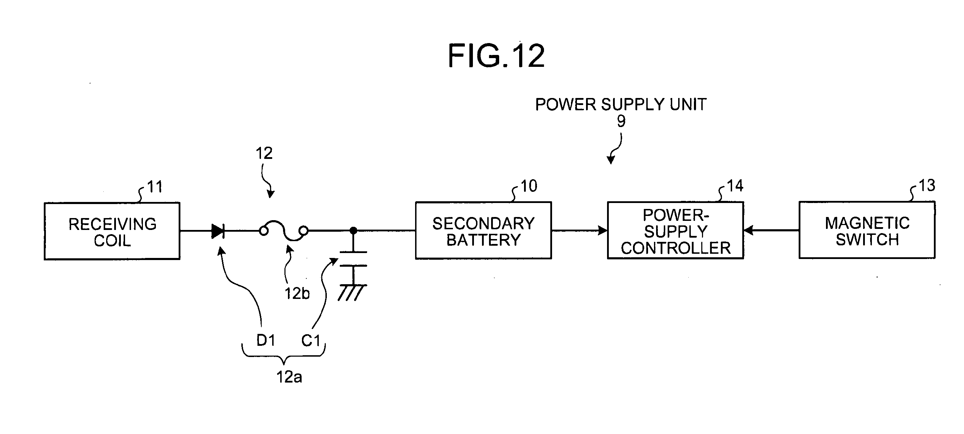

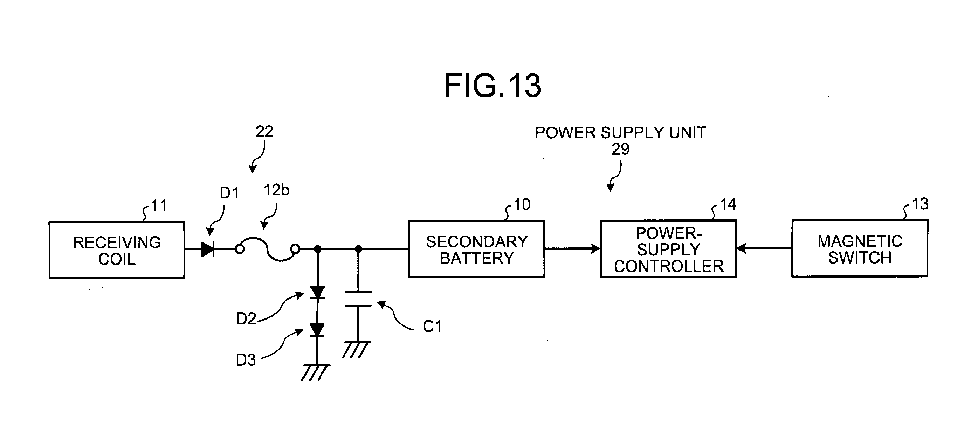

In the first embodiment of the present invention and Modification 1 of the first embodiment, the fuse 12b is arranged between the receiving coil 11 and the diode D1. Alternatively, the fuse 12b may be arranged in a desired position in the conduction path between the secondary battery 10 and the receiving coil 11. Specifically, in the power supply unit 9 of the capsule medical apparatus 1 according to the first embodiment, the fuse 12b may be arranged between the diode D1 and the capacitor C1 as shown in FIG. 12, or between the capacitor C1 and the secondary battery 10. In the power supply unit 29 of the capsule medical apparatus 21 according to Modification 1 of the first embodiment, the fuse 12b may be arranged between the diode D1 and the diode D2 as shown in FIG. 13, or between the diode D2 and the capacitor D2.

In Modification 2 of the first embodiment of the present invention, the fuse 12b is arranged between the switching unit 33 and the capacitor C1. Alternatively, the fuse 12b may be arranged in a desired position as long as it is in the conduction path between the secondary battery 10 and the switching unit 33. For example, the fuse 12b may be arranged between the capacitor C1 and the secondary battery 10.

In the second embodiment of the present invention and Modifications 1 and 2 of the second embodiment, the semiconductor switching device 43 is arranged between the diode D1 and the capacitor C1. Alternatively, the semiconductor switching device 43 may be arranged in a desired position in the conduction path between the secondary battery 10 and the receiving coil 11. For example, the semiconductor switching device 43 may be arranged between the receiving coil 11 and the diode D1 or between the capacitor C1 and the secondary battery 10.

In the first embodiment of the present invention and Modifications 1 and 2 of the first embodiment, a part of the conduction path between the secondary battery 10 and the receiving coil 11 is formed by the fuse 12b. Alternatively, the conduction path between the secondary battery 10 and the receiving coil 11 may be formed by only the fuse 12b. In other words, the fuse 12b forms at least a part of the conduction path between the secondary battery 10 and the receiving coil 11.

In the second embodiment of the present invention and Modifications 1 and 2 of the second embodiment, a part of the conduction path between the secondary battery 10 and the receiving coil 11 is formed by the semiconductor switching device 43. Alternatively, the conduction path between the secondary battery 10 and the receiving coil 11 may be formed by only the semiconductor switching device 43. In other words, the semiconductor switching device 43 forms at least a part of the conduction path between the secondary battery 10 and the receiving coil 11.

In the second embodiment of the present invention and Modifications 1 and 2 of the second embodiment, the semiconductor switching device 43 is a field-effect transistor. The field-effect transistor that is the semiconductor switching device may be any one of PNP-type and NPN-type transistors. The semiconductor switching device 43 is not limited to field-effect transistors, and it may be a PNP-type or NPN-type transistor whose open/closed state is controlled with a base current.

In the first embodiment of the present invention, Modifications 1 and 2 of the first embodiment, the second embodiment of the present invention, Modifications 1 and 2 of the second embodiment, and the third embodiment of the present invention, the magnetic switch 13 is used as the switching unit for switching ON/OFF the power supply unit. However, the switching unit is not limited to this. It suffices that the switching unit for switching ON/OFF the power supply unit may be any device that can detect a control signal from the outside. For example, an optical switch that detects light, such as infrared light, incident in a predetermined pattern from the outside and switches ON/OFF the power supply unit; a ultrasound switch detects an ultrasound signal in a predetermined pattern from the outside and switches ON/OFF the power supply unit; or a wireless switch receives a high-frequency signal, such as a wireless signal, in a predetermined pattern from the outside and switches ON/OFF the power supply unit.

In the first embodiment of the present invention, Modifications 1 and 2 of the first embodiment, the second embodiment of the present invention, Modifications 1 and 2 of the second embodiment, and the third embodiment of the present invention, the receiving coil 11 that converts an external magnetic field to electric power is used as a power input unit that inputs electric power to be supplied to the secondary battery 10. However, the power input unit is not limited to this. It suffices that the power input unit of the capsule medical apparatus receives an external energy and inputs electric power to charge the secondary battery 10. For example, the power input unit may be an input terminal, such as an electric contact, that inputs electric power from an eternal power supply unit.

In the second embodiment of the present invention, a magnetic signal in the predetermined pattern for switching ON/OFF the power supply unit 49 is applied to the magnetic switch 13 to release the connection between the secondary battery 10 and the receiving coil 11. Alternatively, a magnetic signal of a pattern different from that of the magnetic signal for switching ON/OFF the power supply unit 49 may be applied to the magnetic switch 13 to release the connection between the secondary battery 10 and the receiving coil 11. In this case, the magnetic switch 13 receives the magnetic signal in the different pattern and sends instruction information for releasing the connection to the power-supply controller 44. The power-supply controller 44 controls the semiconductor switching device 43 such that it enters the open state based on the instruction information for releasing the connection from the magnetic switch 13, thereby releasing the connection between the secondary battery 10 and the receiving coil 11.

In the second embodiment of the present invention and Modification 1 of the second embodiment, the connection between the secondary battery 10 and the receiving coil 11 is released by applying a magnetic signal or external information in the predetermined pattern to the capsule medical apparatus. Alternatively, the semiconductor switching device 43 can be switched from the open state to the closed state by applying a magnetic signal or external information of a pattern for examination, which is different from the predetermined pattern, may be applied to the capsule medical apparatus. In other words, the power-supply controller 44 may switch the semiconductor switching device 43 from the open state to the closed state to restore the state where the secondary battery 10 and the receiving coil 11 are connected, based on instruction information from the magnetic switch that receives the magnetic signal in the pattern for examination. The power-supply controller 54 may restore the state where the secondary battery 10 and the receiving coil 11 are connected to each other by switching the semiconductor switching device 43 from the open state to the closed state, based on a detection signal from the detector 53 that detects the external information in the pattern for examination. In this case, in the steps of assembling or examining the capsule medical apparatus, the state of the capsule medical apparatus can be switched between the rechargeable state and the charged state. This makes it possible to easily examine the function of inhibiting charging that the capsule medical apparatus has.

In the first embodiment of the present invention, Modifications 1 and 2 of the first embodiment, the second embodiment of the present invention, and Modifications 1 and 2 of the second embodiment, the connection between the secondary battery 10 and the receiving coil 11 is released at any one of the timing at which charging the secondary battery 10 is competed, the timing at which supplying electric power to the function executing unit 15 is started, and the timing at which the predetermined external information is applied to the capsule medical apparatus. Alternatively, a timer that measures the elapsed time from when supplying electric power to the function executing unit 15 is started may be provided to the power-supply controller. In this case, when a predetermined time elapses from when supplying electric power to the function executing unit 15 is started, the power-supply controller releases the connection between the secondary battery 10 and the receiving coil 11.

In the third embodiment, the timer 74b measures the time from when supplying electric power to the function executing unit 15 is started. When the counted value obtained through the counting process, i.e., the elapsed time, reaches the predetermined value, supplying operation power to the function executing unit 15 is stopped. Alternatively, the information obtained by the timer 74b by performing the counting process is not limited to this. It suffices that the information is predetermined information successive during the operation period of the function executing unit 15. For example, the information may be the number of in-vivo images taken by the imaging unit of the function executing unit 15, the number of synchronizing signals, a clock used for operation of the function executing unit 15, or a dividing clock of the clock. In the case where the function executing unit 15 acquires in-vivo information about, for example, temperature, pH, or pressure, the predetermined information may be the number of times such information is acquired or sent. When synchronizing signals are used for acquiring or sending in-vivo information, the synchronizing signals may be counted. In this case, each time the function executing unit takes in-vivo images, the power-supply controller 74 acquires synchronizing signals of in-vivo images from the function executing unit 15, and sequentially counts the synchronizing signals. When the counted value of synchronizing signals that is a result of the counting process of the timer 74b, i.e., the number of in-vivo images taken by the function executing unit 15, reaches a predetermined value, the power-supply controller 74 stops supplying the operation power to the function executing unit 15 to stop operations of the function executing unit 15. In this case, the same functions and effects as those of the third embodiment can be achieved.

In the third embodiment of the present invention, the counted value of the timer 74b is reset when charging the secondary battery 10 is complete. Alternatively, the counted value of the timer 74b may be reset based on a control signal that is input from the outside.

In the third embodiment, the capsule medical apparatus 71 includes the secondary battery 10. Alternatively, the capsule medical apparatus 71 may include a primary battery that is not rechargeable. In this case, the capsule medical apparatus 71 that includes the primary battery is not required to include the receiving coil 11 and the rectifier circuit 12a.

In the third embodiment, the state where operations of the function executing unit 15 are stopped is maintained after the predetermined time elapses from when the function executing unit 15 is started, which limits the number of times the capsule medical apparatus 71 is used is limited to once. Alternatively, the first embodiment, Modifications 1 and 2 of the first embodiment, the second embodiment, Modifications 1 and 2 of the second embodiment, and the third embodiment of the present invention may be appropriately combined. In other words, by providing the fuse 12b and the semiconductor switching device 43 to the capsule medical apparatus 71, the function of releasing the connection between the secondary battery 10 and the receiving coil 11 can be achieved as in the case of any one of the first embodiment, Modifications 1 and 2 of the first embodiment, the second embodiment, and Modifications 1 and 2 of the second embodiment.

In the first embodiment of the present invention, Modifications 1 and 2 of the first embodiment, the second embodiment of the present invention, Modifications 1 and 2 of the second embodiment, and the third embodiment of the present invention, the function executing unit 15 includes two imaging units 4 and 6 that takes in-vivo images of a subject, and the wireless transmitter 7 that transmits the in-vivo images to the outside. Alternatively, the function executing unit of the capsule medical apparatus may include a single imaging unit or at least three imaging units. In this case, the direction in which the function executing unit takes in-vivo images may be different depending on each part whose image is taken. The function executing unit may include an in-vivo information acquiring unit that measures the pH or temperature inside the subject as in-vivo information of the subject, or in-vivo information acquiring unit that detects the state of a tissue as the in-vivo information. Alternatively, the function executing unit may include a mechanism for applying or injecting medicine into the subject or a tissue sampling unit that samples an in-vivo substance of, such as a tissue.

Additional advantages and modifications will readily occur to those skilled in the art. Therefore, the invention in its broader aspects is not limited to the specific details and representative embodiments shown and described herein. Accordingly, various modifications may be made without departing from the scope of the general inventive concept as defined by the appended claims and their equivalents.

## Claims

1. A capsule medical apparatus (1; 21; 31; 41; 51; 61; 71) comprising:
a function executing unit (15) that executes a predetermined function;
a secondary battery (10) that supplies electric power to the function executing unit (15);
a power input unit (11) in which electric power to charge the secondary battery (10) is input; and
a connecting circuit (12; 22; 32; 42) that releasably connects the secondary battery (10) and the power input unit (11) to each other, wherein
the connecting circuit (12; 22; 32; 42) releases a connection between the secondary battery (10) and the power input unit (11) to inhibit charging the secondary battery (10).

2. The capsule medical apparatus (1; 21; 31) according to claim 1, wherein
the connecting circuit (12; 22; 32) includes a fuse (12b) that forms at least a part of a conduction path between the secondary battery (10) and the power input unit (11), and
the fuse (12b) is disconnected when the electric power input by the power input unit (11) is equal to or larger than a predetermined amount to release the connection between the secondary battery (10) and the power input unit (11), which inhibits charging the secondary battery (10).

3. The capsule medical apparatus (21) according to claim 2, wherein the connecting circuit (22) includes at least one diode (D2; D3) that forms a discharging path that branches off the conduction path.

4. The capsule medical apparatus (21) according to claim 3, wherein a sum of forward voltage of at least one diode (D2; D3) is equal to or higher than an open circuit voltage of the secondary battery (10).

5. The capsule medical apparatus (31) according to claim 2, further comprising a charging detecting unit (34) that detects that charging the secondary battery (10) is complete, wherein
the connecting circuit (32) includes a switching unit (33) that forms a discharging path that branches off a conduction path between the power input unit (11) and the fuse (12b),
when charging the secondary battery (10) is not complete, the charging detector (34) opens the switching unit (33) to disconnect the discharging path, and
when charging the secondary battery (10) is complete, the charging detector (34) closes the switching unit (33) to electrically connect the discharging path and the secondary battery (10) through the fuse (12b).

6. The capsule medical apparatus (41; 51; 61) according to claim 1, further comprising a connection control unit (14; 44; 54) that controls releasing the connection between the secondary battery (10) and the power input unit (11) through the connecting circuit (42), wherein
the connecting circuit (42) includes a semiconductor switching device (43) that forms at least a part of a conduction path between the secondary battery (10) and the power input unit (11), and
the connection control unit (44) controls opening and closing the semiconductor switching device (43) to control releasing the connection between the secondary battery (10) and the power input unit (11) via the connecting circuit (42).

7. The capsule medical apparatus (41) according to claim 6, further comprising an instructing unit that issues an instruction for starting supplying the electric power from the secondary battery (10) to the function executing unit (15), wherein
the connection controller (44) controls the semiconductor switching device (43) so that the semiconductor switching device (43) is closed to connect the secondary battery (10) and the power input unit (11) through the connecting circuit (42), and
when the instructing unit (13) issues the instruction for starting supplying the electric power, the connection controller (44) controls the semiconductor switching device (43) so that the semiconductor switching device (43) opens to release the connection between the secondary battery (10) and the power input unit (11).

8. The capsule medical apparatus (51) according to claim 6, further comprising an information detecting unit (53) that detects predetermined external information, wherein
the connection controller (54) controls the semiconductor switching device (43) so that the semiconductor switching device (43) is closed to connect the secondary battery (10) and the power input unit (11) through the connecting circuit (42), and
when the information detecting unit (53) detects the predetermined external information, the connection controller (54) controls the semiconductor switching device (43) so that the semiconductor switching device (43) opens to release the connection between the secondary battery (10) and the power input unit (11).

9. The capsule medical apparatus (51) according to claim 8, wherein the external information is information about any one of magnetic field, light, high frequency, ultrasound, temperature, and pH outside the capsule medical apparatus (51).

10. The capsule medical apparatus (61) according to claim 6, further comprising a charging detecting unit (64) that detects that charging the secondary battery (10) is complete, wherein
when charging the secondary battery (10) is not complete, the connection controller (14) controls the semiconductor switching device (43) so that the semiconductor switching device (43) is closed to connect the secondary battery (10) and the power input unit (11) through the connecting circuit (42), and
when charging the secondary battery (10) is complete, the connection controller (14) controls the semiconductor switching device (43) so that the semiconductor switching device (43) opens to release the connection between the secondary battery (10) and the power input unit (11).

11. The capsule medical apparatus (1; 21; 31; 41; 51; 61; 71) according to claim 1, wherein the function executing unit (15) includes an in-vivo information acquiring unit (4; 6) that acquires in-vivo information of a subject.

12. The capsule medical apparatus (1; 21; 31; 41; 51; 61; 71) according to claim 11, wherein the in-vivo information acquiring unit (4; 6) is an imaging unit that takes in-vivo images of the subject.

13. A method (1; 21; 31) of charging a capsule medical apparatus that includes a function executing unit (15) that executes a predetermined function; a secondary battery (10) that supplies electric power to the function executing unit (15); a power input unit (11) in which electric power to charge the secondary battery (10) is input by receiving an external energy; and a fuse (12b) that connects the secondary battery (10) and the power input unit (11), the method comprising:
charging the secondary battery (10) by applying an external energy to the capsule medical apparatus;
detecting that charging the secondary battery (10) is complete; and
disconnecting the fuse (12b) to inhibit charging the secondary battery (10), when charging the secondary battery (10) is complete.

14. A method (41; 51; 61) of charging a capsule medical apparatus that includes a function executing unit (15) that performs a predetermined function; a secondary battery (10) that supplies electric power to the function executing unit (15); a power input unit (11) in which electric power to charge the secondary battery (10) is input by receiving an external energy; and a semiconductor switching device (43) that connects the secondary battery (10) and the power input unit (11), the method comprising:
charging the secondary battery (10) by applying a predetermined external energy to the capsule medical apparatus;
detecting that charging the secondary battery (10) is complete; and
causing the semiconductor switching device (43) to open to inhibit charging the secondary battery (10), when charging the secondary battery (10) is complete.
